**Europäisches Patentamt**

**European Patent Office**

**Office européen des brevets**

(11) Veröffentlichungsnummer: **0 153 297**

**A 1**

## (12) EUROPÄISCHE PATENTANMELDUNG

(21) Anmeldenummer: 85890026.9

(22) Anmeldetag: 06.02.85

(51) Int. Cl.⁴: **C 02 F 3/28**

(30) Priorität: 06.02.84 AT 367/84

(71) Anmelder: **PFEFFERKORN, Herbert, Arlbergstrasse 101, A-6900 Bregenz (AT)**

(43) Veröffentlichungstag der Anmeldung: 28.08.85 **Patentblatt 85/35**

(84) Benannte Vertragsstaaten: **BE CH DE LI NL**

(72) Erfinder: **PFEFFERKORN, Herbert, Arlbergstrasse 101, A-6900 Bregenz (AT)**

(54) **Einrichtung zur Aufbereitung von organischen Abfallsubstraten in fester und gelöster Form bei gleichzeitiger Gewinnung von Methangas.**

(57) Durch den Einbau von Trennwänden -3- bis -9- mit speziell angeordneten Öffnungen -10- bis -14- sowie -21- und -22- werden die einzelnen Reaktionsräume -100- bis -102- geschaffen, in welchen ein den mikrobiologischen Um- und Abbau fördernder Einfluss erwirkt wird.

Durch die syphonartige Ausbildung des Zulaufrohres -2- kann die Anlage auch gleichzeitig als Hebeanlage betrieben werden.

Dieses neuartige Anlagensystem ermöglicht vor allem, bedingt durch die relativ grossen Rückhalte- und Bearbeitungsräume für Schwimmstoffe und Grundstoffe, die notwendige längere Verweilzeit für den Abbau dieser Feststoffe, im Vergleich zu den leichter und schneller abbaubaren gelösten Stoffen. Die spezielle Formgebung und Anordnung der Überströmungsöffnungen ermöglicht eine selbsttätige Begrenzung der max. Mächtigkeit der Schwimmstoffbereiche. Überschüssige nicht abbaubare Schwimmstoffe werden dabei ebenfalls selbsttätig über den Ablauf -19- ausgeschieden.

Pfefferkorn Herbert

Einrichtung zur Aufbereitung von organisch belasteten Abfallsubstraten in gelöster und fester Form bei gleichzeitiger Gewinnung von Methangas, bestehend aus mehrstufigen Reaktionsräumen, welche vorzugsweise in einem schräg liegenden Baukörper vereinigt und umgehend wärmeisoliert sind sowie mindest einen Zu- und einen Ablauf aufweist.

Einrichtungen dieser Art gehören zum Stand der Technik. Diese Einrichtung stellt im wesentlichen einen in mehrere Reaktionsräume (Kaskaden) unterteilten, schräg liegenden Gärbehälter mit mindestens einem Zulauf und mindest je einem Ablauf für das aufbereitete Gärsubstrat und das in der Anlage produzierte Biogas dar. Der Gärbehälter arbeitet nach dem Verdrängerprinzip und ermöglicht, bedingt durch den Einbau der neuartigen Trennelemente, eine auf die mikrobiellen Bedürfnisse abgestimmte Substratführung in einer Art und Weise, daß während der Verdrängerphase die weitgehend von organischen Ballaststoffen befreite Trägerflüssigkeit in die jeweils nachgeschaltete Kaskade gefördert wird und die schwerer abbaubaren Sink- und Schwimmstoffe in den jeweiligen Kaskaden zurückgehalten werden. Bei jedem Mischvorgang werden die Sink- und Schwimmstoffe durchmischt und gleichzeitig zum Teil in die jeweils davor liegende Kaskade zurückgefördert. Diese Art der Substratführung ermöglicht neben einer Begünstigung des biologischen Ab- und Umbauprozesses auch gleichzeitig eine Seperation der Feststoffe im Gärbehälter, wodurch die erforderliche unterschiedliche Verweilzeit im Gärbehälter von Feststoffen zu gelösten Stoffen erreicht wird.
Durch diese Maßnahme wird die für einen weitgehenden mikrobiologischen Ab-und Umbau der Feststoffe erforderliche verlängerte Verweilzeit, im Vergleich zu den gelösten Stoffen, welche erfahrungsgemäß biologisch schneller umgesetzt werden können, sichergestellt.
Die Ein- und Durchmischungsarbeiten erfolgen in der Regel ohne Zufuhr von Fremdenergie durch Nutzung des Volumenbedarfes der in der Anlage produzierten Gase. Während der

Pfefferkorn Herbert

Einfahrphase und bei niedrig belasteten Anlagen kann durch Zuschaltung eines Gebläses die Umwälzhäufigkeit erhöht werden.Durch die syphonartige Ausbildung der Zulafleitung kann der Gärbehälter auch gleichzeitig als Hebeanlage betrieben werden,wodurch der Ablauf in Bezug zum Zulauf höher angeordnet werden kann. Je nach Formgebung und Einsatzort können die Gäbehälter in Stahlbeton - Kunststoff - Stahl oder auch in verschiedenen Materialkombinationen erstellt werden.

Ein großes Problem stellt in der Praxis in sehr vielen Fällen die für einen Abbauprozess ungünstige Beschaffenheit der mit einem hohen Feststoffanteil durchsetzten Rohsubstrate dar, welche vor allem auf landwirtschaftlichen Betrieben und imBereich der Müllentsorgung sowie auf verschiedenen industriellen Betrieben vorliegt. Diese Gärsubstrate werfen nicht nur bei der Beschickung der Gärbehälter Probleme auf, sondern verursachen in der weiteren Folge derart starke Schwimm- und Sinkschichten, daß die Funktionstüchtigkeit des Gärbehälters soweit beeinträchtigt wird, daß der gesamte Gärprozess zum Erliegen gebracht wird. Des weiteren erschwert der erhöhte Feststoffanteil im Gärsubstrat die Umwälzung und gezielte Führung des Gärsubstrates im Gärbehälter ganz entscheidend. Bei herkömmlichen Anlagen müssen diese Feststoffe vor der Zuführung in die Anlage konditioniert bzw. elliminiert werden,was in der Regel nur mit hohem Energie- und Kostenaufwand möglich ist und grunddessen in den meisten Fällen einen wirtschaftlichen Betrieb derartiger Anlagen verunmöglicht. Die Umwälzung der Gärsubstrate erfolgt heute im wesentlichen nach folgenden Methoden:

1) Umpumpen der Gärmasse über extern angeordnete Pumpen
2) Umwälzung durch intern angeordnete Rühr- und Mischeinrichtungen.
3) Umwälzung durch Gaseinpressung in die Gärmasse.
4) Umwälzung mittels Verdrängersystem.

Unter Einbeziehung der chinesischen Kleinanlagen stellen heute die nach dem Verdrängersystem arbeitenden Anlagen das mit Abstand größte Kontingent dar. Beim Verdrängersystem benötigt man mindestens 2 getrennte Räume, welche gegenseitig kommunizierend verbunden sind. Durch Verriegelung des Gasabganges aus dem ersteren Raum, verdrängt das an der Oberfläche des Flüssigkeitsspiegels ansammelnde Gas, die Flüssigkeit über die kommunizierende Verbindung in den zweiten bzw. höher liegenden und gasseitig offenen Behälter. Durch Ablassen des verdichteten Gaspolsters strömt die nach oben verdrängte Flüssigkeit, dem Gesetz der Schwerkraft folgend, wieder in den ersteren Raum zurück. Nach Verriegelung des Gasabganges erfolgt der Verdrängerprozess von Neuem. Als Kraftquelle wird das in der Anlage gewonnene Biogas verwendet. Diese Anlagen sind jedoch nur bis zu einem mäßigen Anteil von Feststoffen im Gärsubstrat funktionstüchtig. Ein höherer Anteil an Feststoffen verursacht eine erschwerte Umwälzung und fördert die Sink- und Schwimmdeckenbildung dermaßen, daß diese Anlagen funktionsuntüchtig werden und in der weiteren Folge die aufwendige Räumung dieser Sink- und Schwimmschichte notwendig macht.

In der österr. PS. Nr. 361885 und in der europäischen Patentanmeldung, Anmeldenummer 79810180.4 werden nach dem Verdrängerprinzip arbeitende Gärbehälter beschrieben, welche eine Hauptgärkammer - eine Nachgärkammer und einen Gassammelraum aufweisen. Die Raumunterteilung wird dabei durch den Einbau von Zwischendecken bewerkstelligt, wobei der Gassammelraum und der Nachklärraum niveaugleiche Decken aufweisen. Die hydraulische Verbindung erfolgt über den Einbau von speziell angeordneten Schächten. Diese Anlagen weisen in der Praxis bei Vorhandensein von entsprechenden Feststoffmengen im Gärsubstrat Probleme durch Bildung von starken Schwimm- und Sinkschichten auf, welche nach Erreichen einer entsprechenden Mächtigkeit die Funktion der Anlagen zum Erliegen bringen. Vorallem im Bereich der Nachklärräume bilden sich auf Grund der fehlenden Bekämpfungsmöglichkeit starke Schwimmstoffschichten, welche eine kon-

Pfefferkorn Herbert

tinuierliche Funktionstüchtigkeit dieser Anlagen verunmöglichen.

Dieses Verfahrenssystem verursacht, bedingt durch die komplizierte Bauart und dem Vorhandensein von einem hohen Anteil von ungenützten Gasräumen, neben hohen Erstellungskosten auch höhere Wärmeverluste über das Bauwerk selbst, da infolge des relativ hohen ungenutzten Gasraumanteiles auch die Gesamtoberfläche des Gärbehälters entsprechend ansteigt.

Die Erfindung stellt eine unmittelbare Weiterentwicklung dieser nach dem Verdrängerprinzip arbeitenden Anlagen dar und erschließt, bedingt durch die wesentlich vereinfachte und kostengünstigere Konzeption bei gleichzeitig verbesserter Führung - Durchmischung und Heben des Gärsubstrates, neue Einsatzgebiete vorallem in landwirtschaftlichen und industriellen Bereichen der Entsorgung bzw. Aufbereitung von organischen Abfällen,was vorschlagsgemäß durch den Einbau von Wandscheiben in einen schräg liegenden Behälter sowie der syphonartigen Ausbildung des Zulaufes erreicht wird. Die Aufgaben werden erfindungsgemäß wie folgt gelöst:

Durch den Einbau der Trennwände -3-, -4-, -5-, -6-, -7-, -8-, -9- mit den anteiligen Öffnungen -10-, -11-, -12-, -13-, -14-, -21-, -22- werden Kaskaden (Reaktionsräume) -100-, 101-, -102- geschaffen.

Durch den Einbau der Trennwände -3- bis einschließlich -9- mit den anteiligen Öffnungen -11-, -12-, -13-, -14-, -21-, -22- in einen schräg liegenden Baukörper -1- werden die abgestuften Reaktionsräume -100-, 101-, -102- geschaffen.

Als Baukörper können dabei z.B. kubische - rechteckförmige und kreiszylinderförmige Bauformen zum Einsatz gebracht werden. Die Beschickung der Anlage erfolgt über einen syphonartigen Zulaf, der in die an der tiefsten Stelle liegende Kaskade -100- zugeführt wird und der syphonartige Ablauf erfolgt von der letzten in der Reihe angeordneten Kaskade -102-. Durch Verriegelung der Gasüber-

Pfefferkorn Herbert

Strömungsleitung -16- mittels dem Absperrorgan -17- oder der mit Gärabstrat gefüllten Überströmungsleitung -23-, sammelt sich das in den an der Einlaufseite liegenden Kaskaden -1oo-, -1ooa- produzierte Gas an der Oberfläche des Flüssigkeitsspiegels und verdrängt im gleichen Maße die Gärflüssigkeit in die angrenzenden Reaktionsräume, bis die max. Druckdifferenz -x- zwischen den Flüssigkeitsspiegeln -U- und ' -O- erreicht ist. Die Begrenzung des max. gedrückten Flüssigkeitsspiegels -U- erfolgt über die Öffnung -10- in Trennwand -4- bzw. durch die Höhenlage des horizontal liegenden Gasüberströmungsrohres -23-. In beiden Fällen strömt das verdichtete Gaspolster bzw. ein Teil dessen über die angeführten Entlastungsöffnungen in die dahinter liegenden Reaktionsräume -1o1- ab. Im Zuge der Gasüberströmung wird die Sperrflüssigkeit aus dem Überströmungsrohr -23- ausgestoßen und der Gasübergang -23- bleibt bis zum Abschluß des Mischvorganges in den Reaktionsräumen, bedingt durch die verzögerte Wiederauffüllung des Gasüberströmungsrohres mit Gärflüssigkeit auf Grund der reduzierten Einlauföffnung im Füllstutzen -24-, offen. Da die Gasabgangsleitung -25- im lichten Querschnitt ebenfalls kleiner als die Überströmungsleitung -23- gehalten wird und gleichzeitig kein Gasraum in den nachgeschalteten Reaktionsräumen vorliegt, erfolgt mit Beginn des Überströmen des Gases ein momentaner Druckausgleich im Gasbereich der einzelnen Kaskaden, wodurch gleichzeitig das Zurückströmen der vorgängig nach oben verdrängten Gärflüssigkeit in die jeweils tieferliegende Kaskade eingeleitet wird. Durch den Einbau von zusätzlichen Mengenregelorganen -17'- in die Gasüberströmungsleitung, kann das Zurückströmen der Gärflüssigkeit zwischen den einzelnen Kaskaden zeitlich unabhängig beziehungsweise im reduzierten Maße durchgeführt werden. Des weiteren wird soweit nur ein Absperrorgan -17- in der Gasüberströmungsleitung -16- eingebaut ist, die Rückströmungsmenge zwischen den einzelnen Kaskaden durch eine entsprechende Fließquerschnittsvorgabe zwischen den Trennwänden und deren Strömungsöffnungen derart beeinflußt, daß

0153297

Pfefferkorn Herbert

die Rückströmungszeiten je nach Erfordernis in einzelnen Kaskaden verzögert werden. Nach Abschluß des Mischvorganges wird das Absperrorgan -17- wieder geschlossen bzw. die Gasüberströmungsleitung -23- wieder mit Gärflüssigkeit gefüllt, wodurch der Verdrängungsprozess wieder von Neuem beginnt. Bei der Anordnung der Verbindungsöffnungen -1o- und -13- wurde vorausgesetzt, daß ein Großteil der im Gärsubstrat vorhandenen Feststoffe auf Grund des unterschiedlichen spezifischen Gewichtes sich während der Verdrängerzeit in Form von Sink- und Schwimmschichten in den einzelnen Kaskaden ansammeln und auf Grund der speziellen Anordnung diese Verbindungsöffnungen im Bereich zwischen diesen mit Feststoffen angereicherten Schichten diese Feststoffe in der jeweiligen Kaskade zurückgehalten werden und vorwiegend nur Trägerflüssigkeit verdrängt wird. Des weiteren wird durch diese spezielle Situierung der Verbindungsöffnungen eine Begrenzung der max. Mächtigkeit der Schwimmdecken erreicht und die nicht abbaubaren überschüssigen Schwimmstoffe werden über den Ablauf -19- nach außen abgeführt. Damit in der letzten Kaskade -1o2- eine Schwimmdeckenbildung nur im geringen Umfang erfolgen kann, wurde der Ablauf -19- so hoch angelegt, daß eine annähernde Vollfüllung der Kaskade sichergestellt ist und andererseits wird eine Teilmenge des überströmenden Gases bei jedem Mischvorgang über die Verbindungsleitung -18- in den oberen Flüssigkeitsbereich eingepreßt, wodurch eine starke Auflockerung und Zerstörung bzw. der Abtransport von Schwimmdeckenansätzen sichergestellt ist. Der überschüssige Grundschlamm hingegen wird perjodisch je nach Erfordernis über den Abzug -2o- aus der tiefsten Stelle der Anlage abgelassen. Die Anordnung der Trennwände -3- bis einschließlich -9- erfolgt mit ihren Verbindungsöffnungen in Abstimmung auf das aufzubereitende Substrat, in einer Art und Weise, daß die für einen funktionstüchtigen mikrobiellen Abbauprozess jeweils möglichst günstigsten Randbedingungen aus physikalischer und mikrobieller Sicht gegeben sind. Die gezielte Substratführung wird dabei vorwiegend über die Situierung und der

Pfefferkorn Herbert

entsprechenden Fließquerschnittvorgabe sichergestellt, wobei bei Gärsubstraten mit starker Schwimmdeckenbildung während des Mischvorganges die Trägerflüssigkeit vom bodennahen Bereich der jeweils höher liegenden Kaskade in die Schwimmdecke der tieferliegenden Kaskade eingemischt wird und bei Gärsubstraten mit starken Sinkschichtenbildungen die Trägerflüssigkeit in umgekehrter Weise vom deckennahen Bereich der höher liegenden Kaskade in den Sinkschlammbereich der tiefer liegenden Kaskade eingemischt wird. Durch die Anordnung der schräg liegenden Decken- und Bodenplatten wird die Bekämpfung der Schwimm- und Sinkschichten weiter, durch Einwirken von zusätzlichen mechanischen Scheerkräften in die Schwimmdecke während der Befüllungsphase sowie des erleichterten Rücktransportes der Sinkschichte in die jeweils tiefer liegende Kaskade während des Mischvorganges, begünstigt. Ein weiterer entscheidender Vorteil liegt darin, daß durch die Anordnung eines syphonartigen Zulafes -2-, in welchem ein Rückströmen von Gärsubstraten in die Zulafleitung während der Verdrängerphase durch die Anordnung der Mündungsöffnung knapp über der Ruhespiegellinie - N- unterbunden wird und ein gleichzeitiges Hochfördern des Gärsubstrates in den höher liegenden Ablauf -19- erfolgt. Dasselbe Ziel wird auch durch den Einbau eines Absperrorganes -27- in die Beschikkungsleitung -2'- erreicht, wobei dieses Absperrorgan nur kurzfristig während der Beschickung, jeweils direkt nach dem Mischvorgang geöffnet wird.

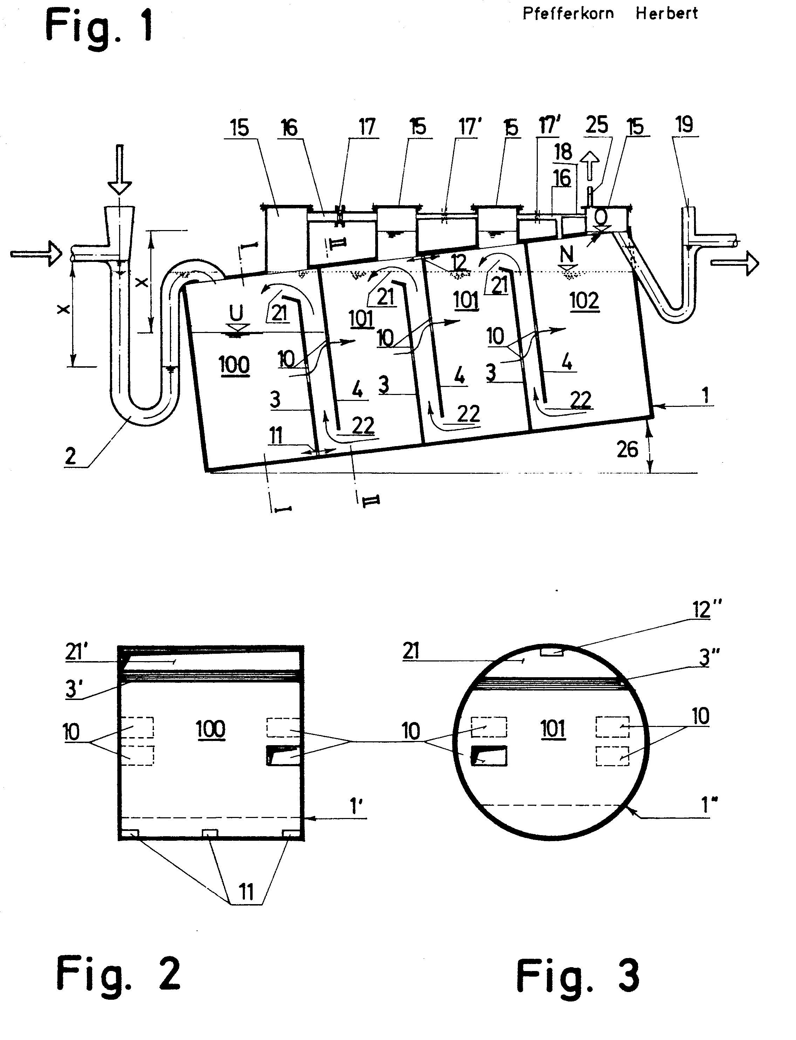

Die Figur 1 stellt einen längs geführten Vertikalschnitt durch eine Anlage dar, in welcher vorwiegend Gärsubstrate mit Neigungen zur Schwimmdeckenbildung aufbereitet werden.

Figur 2 und 3 stellen nach den Linien I und II geführte Regelquerschnitte einer rechteck- sowie kreiszylinderförmigen Aufbereitungsanlage dar, in welcher auch die in die Trennwände eingebauten Verbindungsöffnungen angeführt sind.

0153297

Pfefferkorn Herbert

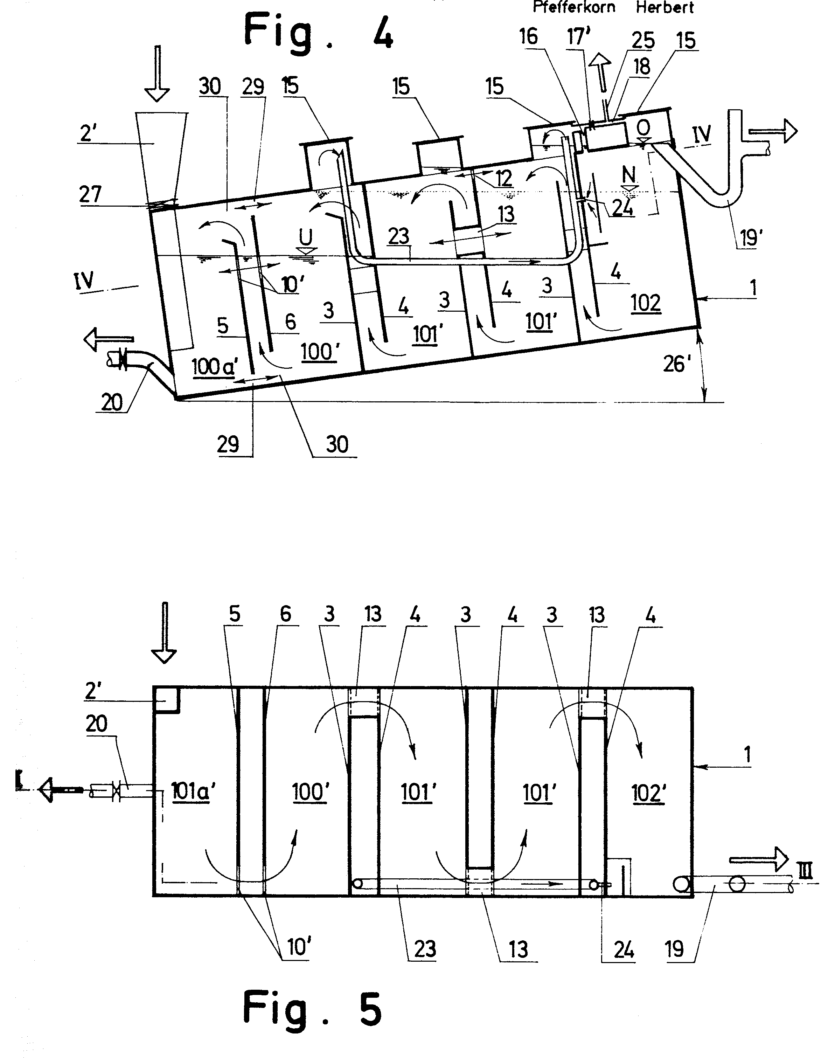

Die Figur 4 stellt einen vertikalen Längsschnitt nach der in Figur 5 geführten Linie III durch eine Anlage in welcher die Hauptgärkammer in 2 Kaskaden unterteilt und die Gasabgangsregelung aus der Hauptgärkammer über die druckabhängige, syphonartige Gasüberströmungsleitung erfolgt.

Figur 5 stellt einen nach der Linie IV durch die unter Figur 4 dargestellten Anlage geführten parallel zur Deckenebene verlaufenden Schnitt dar, in welchem die Substratführung während der Verdrängerphase dargestellt wird.

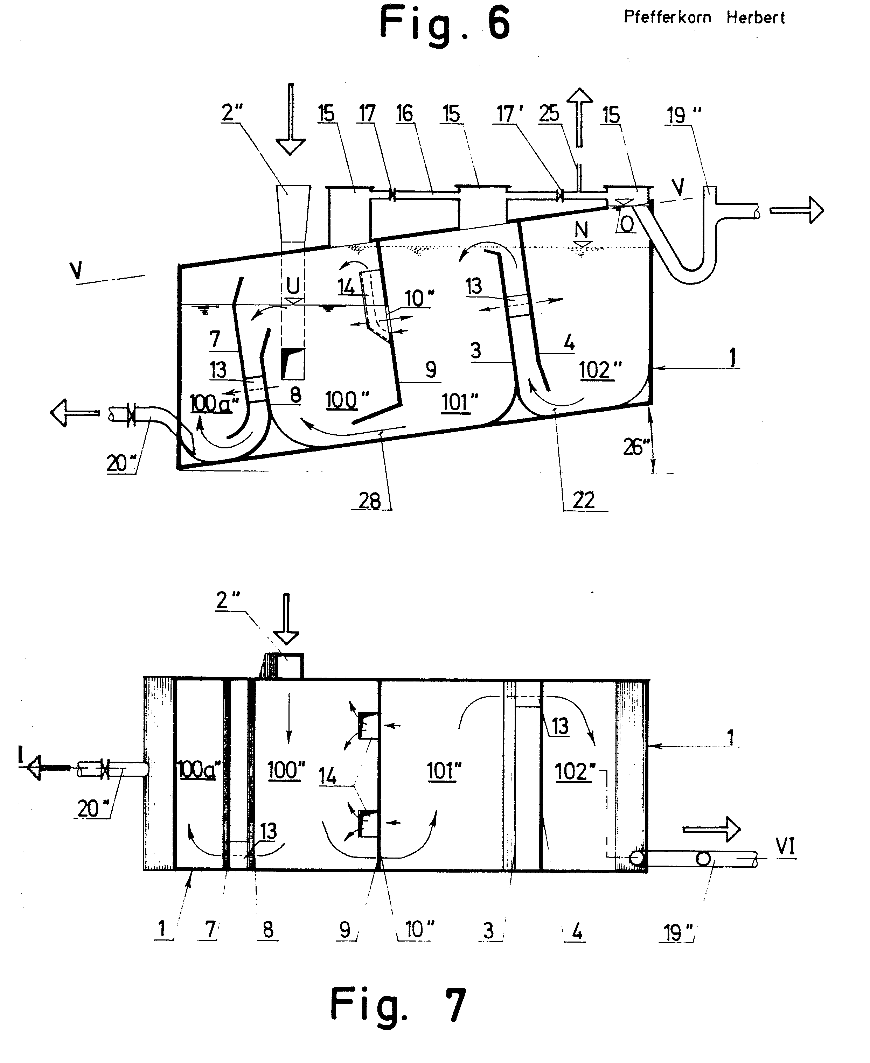

Figur 6 stellt einen nach der Linie VI in Figur 7 dargestellten Anlage, geführten vertikalen Längsschnitt dar, in welchem eine Anlage für die Aufbereitung von Gärsubstrat mit einem hohen Anteil von Sinkstoffen dargestellt ist, wobei die Hauptgärkammer ebenfalls aus 2 Kaskaden -100"- und -1ooa"- besteht und die tieferliegende Kaskade -1ooa"- als Sinkstoff-Nachbehandlungs- und Pufferraum konzeptiert ist. Beim Mischen wird bei diesem Anlagentyp ein Großteil der in die Kaskaden -1oo"- und -1ooa"- zurückströmenden Gärflüssigkeit in den bodennahen Sinkstoffbereich eingemischt. Die untere Kante der in die Trennwand -7- und -8- eingebauten Öffnung -13- begrenzt den max. Sinkstoffstand in der Kaskade -1oo"- und leitet die überschüssigen Sinkstoffe zur Nachbehandlung in die Kaskade -1ooa"- ab, von wo sie intervallmäßig je nach Bedarf über die Entnahmevorrichtung -2o- abgelassen werden. Damit in der Hauptgärkammer -1oo"- auch eine begrenzte Schwimmstoffbehandlung möglich ist, sind die Rückführschächte -14-, welche an der unteren Seite zur Kaskade -1o1"- und an der oberen Seite zur Hauptgärkammer -1oo"- offen sind, angeordnet. Unter der Annahme, daß sich die in der Mischphase aufgewirbelten Sinkstofe während der Verdrängerphase, bedingt durch Sedimentationserscheinungen, aug der Bodenplatte ansammeln und in der weiteren Folge ein Überströmen in die agrenzende Kaskade

0153297

Pfefferkorn Herbert

-1o1"- über die zwischen Bodenplatte und Trennwand -9- befindliche Öffnung -28- verunmöglichen, erfolgt die Verdrängung aus der Hauptgärkammer, über die in der Trennwand eingebaute Öffnung -1o"-, welche auch gleichzeitig die Begrenzung der max. Schwimmschicht in der Hauptgärkammer darstellt.

Figur 7 stellt einen nach der Linie V parallel zur Decke verlaufenden Schnitt, durch die unter Figur 6 angeführten Anlage, dar. Die Beschickung erfolgt bei diesem Anlagentyp in die höher liegende Kaskade -1oo"- der Hauptgärkammer.

Pfefferkorn Herbert

Patentansprüche

1. Einrichtung zur Aufbereitung von organisch belasteten Abfallsubstraten in gelöster und fester Form bei gleichzeitiger Gewinnung von Methangas, bestehend aus mehrstufigen Reaktionsräumen, welche vorzugsweise in einem schräg liegenden Baukörper vereinigt und wärmeisoliert sind, mindest einen Zu- und Ablauf aufweisen, dadurch gekennzeichnet, daß die einzelnen Reaktionsräume -1oo-, -1o1-, -1o2- durch den Einbau von Trennwänden -3- bis einschließlich -9- mit Öffnungen -1o- bis -14- sowie -21-, -22- in einer Art und Weise erfolgen, daß während der Verdrängerphase ein bestimmter Anteil der Sink- und Schwimmstoffe in dem jeweiligen Reaktionsraum zurückgehalten werden und die dazwischen liegende Trägerflüssigkeit in den jeweils nachgeschalteten Reaktionsraum verdrängt wird und beim Rückmischen der verdrängten Gärmasse der jeweils oben liegende Reaktionsraminhalt in den unten liegenden Bereich des vorgeschalteten Reaktionsraumes eingemischt oder in umgekehrter Weise die unten liegenden Sinkstoffe in den oberen Bereich der vorgeschalteten Kaskade eingemischt werden sowie der Zulauf -2- syphonartig ausgebildet ist.

2. Einrichtung nach Anspruch 1 dadurch gekennzeichnet, daß das Trennscheibenpaar bestehend aus den in einem vorgegebenen Abstand eingebauten Wandscheiben -3- und -4- und die Scheibe -3- in Deckennähe die Öffnung -21- und die Scheibe -4- die Öffnung 22 in Bodennähe aufweist.

3. Einrichtung nach Anspruch 1 und 2 dadurch gekennzeichnet, daß die Öffnungen -1o- in den Wandscheiben -3- und -4- vorzugsweise jeweils an die Außenwände anschließen und bei jedem darauffolgenden Kaskadenübergang die Seiten wechseln und in der Höhenlage so angeordnet sind, daß Öffnung in Scheibe -4- höher angeordnet ist.

Pfefferkorn Herbert

4. Einrichtung nach Anspruch 1 und 2 dadurch gekennzeichnet, daß in das Trennscheibenpaar -3- und -4- eineoder mehrere röhrenförmige Verbindungen -13- eingebaut sind.

5. Einrichtung nach Anspruch 1 bis 2 dadurch gekennzeichnet, daß in die Trennwand -9- eine oder mehrere bodennahe Öffnungen -28- sowie eine oder mehrere knapp unter dem max. gedrückten Flüssigkeitsspiegel liegende Öffnungen -1o- mit angeschlossenen Rückförderschächten -14- eingebaut sind.

6. Einrichtung nach Anspruch 1 bis 2 dadurch gekennzeichnet, daß das Trennscheibenpaar -7- und -8- an der Deckenseite unterschiedlich hohe Öffnungen afweist, wobei die tieferliegende Öffnung bzw. Öffnungen in Trennscheibe -8- bis unter dem max. gedrückten Spiegel -U- reichen und die Trennscheibe -7- in Bodennähe eine oder mehrere Öffnungen sowie eine oder mehrere rohrförmige das Trennscheibenpaar -7- und -8- verbindende Öffnungen aufweist.

7. Einrichtung nach Anspruch 1 bis 6 dadurch gekennzeichnet, daß der Zulaufschacht -2- mit einem Absperrorgan -27- versehen ist.

8. Einrichtung nach Anspruch 1 bis 7 dadurch gekennzeichnet, daß die Gasüberströmungsleitung -23- syphonartig mit einer in der Höhe der maximal gedrückten Spiegelebene -U- horizontal verlaufenden Rohrabschnitt und einen im Vergleich zum Gasüberströmungsrohr -23- kleineren lichten Fließquerschnitt aufweisenden Füllstutzen -24-, welcher unter der Ruhespiegellinie -N- in Kaskade -1o2- mündet und die beiden offenen Rohrenden der Leitung -23- über max. Füllspiegellinien

0153297

Pfefferkorn Herbert

-N- und -O- der jeweiligen Reaktionsräume -1oo-, -1oo'- und -1o1-, -1o1'- münden.

9. Einrichtung nach Anspruch 1 bis 8 dadurch gekennzeichnet, daß die Gasüberströmungsleitung -16- unter der Decke der Kaskade -1o2- mündet und eine Verbindungsleitung -18- mit reduziertem Strömungsquerschnitt zum Schacht -15- der Kaskade -1o2- aufweist.

1o. Einrichtung nach Anspruch 1 bis 9 dadurch gekennzeichnet, daß in der Gasüberströmungsleitung-16- ein oder mehrere Mengenregelorganze -17'- eingebaut sind.

Pfefferkorn   Herbert

# Fig. 1

# Fig. 2

# Fig. 3

0153297
Pfefferkorn  Herbert

# Fig. 4

# Fig. 5

0153297
Pfefferkorn Herbert

# Fig. 6

# Fig. 7

**Europäisches Patentamt**

**EUROPÄISCHER RECHERCHENBERICHT**

0153297
Nummer der Anmeldung

EP 85 89 0026

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 013 538 (R. MANAHL) * Seite 17, Zeile 32 – Seite 19, Zeile 24 * | 1 | C 02 F 3/28 |
| A | WO-A-8 001 286 (F. PFULG) * Seite 8, Ansprüche 1, 2 * | 1 | |
| A | CH-A- 436 147 (G. DUNAND et al.) * Spalte 3, Zeilen 10-15; Spalte 4, Zeilen 40-44 * | 2,3,5, 6 | |
| P,A | DE-A-3 230 010 (K. IWAN) * Titelseite, Zusammenfassung * | 1 | |

RECHERCHIERTE SACHGEBIETE (Int. Cl.4)

C 02 F
C 12 M

Der vorliegende Recherchenbericht wurde für alle Patentansprüche erstellt.

| Recherchenort DEN HAAG | Abschlußdatum der Recherche 14-05-1985 | Prüfer TEPLY J. |
|---|---|---|